# EUROPEAN PATENT APPLICATION

(11) **EP 4 674 968 A1**
(43) Date of publication of application: **07.01.2026**
(21) Application number: 25745378.7
(22) Date of filing: 24.01.2025
(51) Int. Cl.: C12N 15/70, C12N 9/88, C12P 13/00

(54) **RECOMBINANT MICROORGANISM COMPRISING NOVEL LYSINE DECARBOXYLASE AND PRODUCING PENTAMETHYLENEDIAMINE AND METHOD FOR PRODUCING PENTAMETHYLENEDIAMINE BY USING SAME**

(30) Priority: 25.01.2024 KR 20240011856
(71) Applicant: LG Chem, Ltd., Seoul 07336 (KR)
(72) Inventor: EOM, Hyunjoo, Daejeon 34122 (KR); JUNG, Kwonsu, Daejeon 34122 (KR); KIM, Seonwoo, Daejeon 34122 (KR); PARK, Jin Hwan, Daejeon 34122 (KR); PARK, Si Jae, Daejeon 34122 (KR); SON, Jina, Daejeon 34122 (KR); LEE, Ji Yeon, Daejeon 34122 (KR); LEE, Haeyoung, Daejeon 34122 (KR)
(74) Representative: Goddar, Heinz J.
(86) International application number: PCT/KR2025/001424
(87) International publication number: WO 2025/159556

(57) **Abstract**

The present description relates to a recombinant microorganism having production activity of pentamethylenediamine (PMDA), which comprises an *E. coli* Nissle-derived decarboxylase enzyme protein, and uses thereof.

## Description

### [TECHNICAL FIELD]

### Cross-reference to related applications

The present application claims the benefit of the priority based on Korean Patent Application No. 10-2024-0011856 filed on January 25, 2024, and the entire contents disclosed in the documents of the corresponding Korean patent application are incorporated as a part of the present description.

The present description relates to a recombinant microorganism having production activity of pentamethylenediamine (PMDA), which comprises an *E. coli* Nissle-derived decarboxylase enzyme protein, and uses thereof.

### [BACKGROUND ART]

PMDA (Pentamethylenediamine) is a C5 diamine, which is a substance used as a monomer of nylon PA56, PA510. PDMA can be produced by a biological method through enzyme conversion of lysine decarboxylate using an amino acid, lysine, as a raw material. There are largely two main types of lysine decarboxylase studied so far, CadA and LdcC, and LdcC is known to show a tendency to resist high pH better than CadA.

For production of the PMDA, an *E. coli* strain was used. However, it is necessary to identify a suitable strain to improve the efficiency of PMDA production in industrial processes. Non-pathogenic *E. coli* Nissle (EcN) has been found to have no enterotoxins, cytotoxins, pathogenic adhesion factors, serum resistance, antibiotic resistance genes, and the like, and has been known as a relatively safe host strain in LMO (Living Modified Organism) issue, and therefore, when EcN is used as a host strain, PMDA can be safely produced in the LMO issue.

### [DISCLOSURE]

### [TECHNICAL PROBLEM]

Under the above background, the excellent PMDA production activity of the recombinant microorganism, in which *E*. *coli* Nissle-derived lysine decarboxylase (LDC) was introduced, was confirmed, thereby completing the present invention.

One embodiment of the present description provides a recombinant microorganism, comprising (and/or expressing) *E. coli* Nissle microorganism-derived lysine decarboxylase enzyme protein, and having production activity of pentamethylenediamine (PMDA).

Another embodiment of the present description provides a recombinant vector, comprising a polynucleotide encoding *E. coli* Nissle microorganism-derived lysine decarboxylase enzyme protein.

Other embodiment of the present description provides a composition for producing pentamethylenediamine, comprising the recombinant microorganism, *E*. *coli* Nissle microorganism-derived lysine decarboxylase enzyme protein, a polynucleotide encoding the enzyme protein, and/or the recombinant vector.

Other embodiment of the present description provides a use of a recombinant microorganism comprising (and/or expressing) *E. coli* Nissle microorganism-derived lysine decarboxylase enzyme protein for producing pentamethylenediamine.

Other embodiment of the present description provides a use of a recombinant vector comprising a polynucleotide encoding *E. coli* Nissle microorganism-derived lysine decarboxylase enzyme protein for producing pentamethylenediamine.

Other embodiment of the present description provides a use of a recombinant microorganism comprising (and/or expressing) *E. coli* Nissle microorganism-derived lysine decarboxylase enzyme protein for producing pentamethylenediamine, and/or a recombinant vector comprising a polynucleotide encoding *E. coli* Nissle microorganism-derived lysine decarboxylase enzyme protein, for preparing a composition for producing pentamethylenediamine.

Other embodiment of the present description provides a method for producing pentamethylenediamine, comprising reacting the recombinant microorganism with a substrate.

### [TECHNICAL SOLUTION]

One embodiment of the present description provides a recombinant microorganism, comprising an *E. coli* Nissle microorganism-derived lysine decarboxylase (LDC) enzyme protein, and/or a polynucleotide encoding *E. coli* Nissle microorganism-derived lysine decarboxylase enzyme protein.

Another embodiment of the present description provides a method for preparing a recombinant microorganism, having production activity of pentamethylenediamine, comprising introducing *E. coli* Nissle microorganism-derived lysine decarboxylase enzyme protein, a polynucleotide encoding the protein, and/or a recombinant vector comprising the polynucleotide into a microorganism (host cell).

The lysine decarboxylase enzyme protein may be expressed by LdcC gene and/or CadA gene, and the polynucleotide encoding *E. coli* Nissle microorganism-derived lysine decarboxylase enzyme protein may be LdcC gene and/or CadA gene of *E. coli* Nissle, but not limited thereto.

Other embodiment of the present description provides a recombinant vector, comprising a polynucleotide encoding *E. coli* Nissle microorganism-derived lysine decarboxylase enzyme protein.

Other embodiment of the present description provides a composition for producing pentamethylenediamine, comprising at least one selected from the group consisting of the recombinant microorganism, *E. coli* Nissle microorganism-derived lysine decarboxylase enzyme protein, a polynucleotide encoding the protein, and a recombinant vector comprising the polynucleotide.

Other embodiment of the present description provides a method for producing pentamethylenediamine, comprising reacting the recombinant microorganism with a substrate.

The recombinant microorganism can have excellent pentamethylenediamine production activity by comprising (or expressing or introducing) *E. coli* Nissle microorganism-derived lysine decarboxylase (LDC) enzyme protein and/or a polynucleotide encoding the protein, compared to a microorganism that does not comprise the same (for example, non-modified or wild-type microorganism).

Hereinafter, the present application will be described in more detail.

### Recombinant microorganism having pentamethylenediamine production activity

One embodiment of the present description provides a recombinant microorganism, comprising an *E*. *coli* Nissle microorganism-derived lysine decarboxylase (LDC) enzyme protein or a polynucleotide encoding *E. coli* Nissle microorganism-derived lysine decarboxylase enzyme protein. The recombinant microorganism may express the *E. coli* Nissle microorganism-derived lysine decarboxylase (LDC) enzyme protein.

More specifically, the recombinant microorganism may be introduced with at least one selected from the group consisting of *E. coli* Nissle microorganism-derived lysine decarboxylase (LDC) enzyme protein, a polynucleotide encoding the protein, and a recombinant vector comprising the polynucleotide. The recombinant microorganism may have pentamethylenediamine (PMDA) production activity and/or have increased pentamethylenediamine production ability compared to a microorganism not comprising the *E. coli* Nissle microorganism-derived lysine decarboxylase enzyme protein (non-modified or wild-type microorganism).

Other embodiment of the present description provides a method for preparing a recombinant microorganism, having pentamethylenediamine production activity, comprising introducing *E. coli* Nissle microorganism-derived lysine decarboxylase enzyme protein, a polynucleotide encoding the protein, and/or a recombinant vector comprising the polynucleotide into a microorganism (host cell) (for example, *Escherichia* sp. microorganism (specifically, *E. coli* Nissle, *E. coli* WL3110, *E. coli* XL1-Blue, *E*. *coli* BL21 (DE3)), *Corynebacterium* sp. microorganism (specifically, *Corynebacterium glutamicum*) or *Saccharomyces* sp. microorganism (specifically, *Saccharomyces cerevisiae*)).

The recombinant microorganism may be introduced with a polynucleotide encoding *E. coli* Nissle microorganism-derived lysine decarboxylase enzyme protein (for example, LdcC gene and/or CadA gene). The lysine decarboxylase enzyme protein may be expressed by LdcC gene and/or CadA gene, but not limited thereto.

In the present description, the term "activity enhancement" or "enhanced activity" refers not only to an effect beyond the original function of a protein through de novo introduction or increase in its intrinsic activity, but also to a state in which the activity of a microorganism is increased after manipulation compared to that before manipulation. Such manipulation includes, but is not limited to: increased gene expression, increased endogenous gene activity, amplification of endogenous genes by internal or external stimuli, deletion of inhibitory regulatory elements of gene expression, increase in gene copy number, introduction of foreign genes, modification of regulatory sequences, replacement or mutation of promoters, and/or enhancement of enzyme activity through mutation within the gene itself.

In the present description, enhancement of the activity of a protein or enzyme (e.g., E. coli Nissle microorganism-derived lysine decarboxylase) may be achieved through various methods well known in the art. These methods include, without limitation: additional insertion of a polynucleotide comprising a nucleotide sequence encoding the relevant enzyme (or protein) into the chromosome, increasing the copy number of the gene by introducing the polynucleotide into a vector system, replacing the promoter capable of driving expression with a stronger promoter, introducing mutations into the promoter, and mutating the gene so that it encodes a variant enzyme (or protein) with enhanced activity.

The *E. coli* Nissle microorganism-derived lysine decarboxylase enzyme protein may be a foreign enzyme protein. In the present description, "foreign" may refer to what is not inherently present, and is introduced from outside by a common method mentioned above such as a recombinant method and the like, and may mean what is derived from another heterologous or homologous strain or cell.

The recombinant microorganism may have the enhanced activity of *E. coli* Nissle microorganism-derived lysine decarboxylase enzyme.

The nucleic acid (gene) sequence or amino acid sequence provided in the present description may include those modified by conventional mutagenesis, for example, direct evolution and/or site-directed mutagenesis, and the like, within a range in which their original function or targeted function are maintained. In one embodiment, that a polynucleotide or polypeptide "comprises, has a specific nucleic acid sequence or amino acid sequence, or consists of a specific nucleic acid sequence or amino acid sequence" may mean that the polynucleotide or polypeptide (i) essentially comprises the specific nucleic acid sequence or amino acid sequence, or (ii) consists of a nucleic acid sequence or amino acid sequence having identity of 60% or more, 70% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more to the specific nucleic acid sequence or amino acid sequence, and maintains the original function and/or targeted function. In the present description, the targeted function may refer to a function of increasing or giving production ability of pentamethylenediamine of a microorganism.

The nucleic acid sequence described in the present description may have various modifications in the coding region within a range without changing the amino acid sequence and/or function of a protein expressed from the coding region, considering the codon preferred in a microorganism that is intended to express the protein due to degeneracy of the codon.

In the present description, the term "identity" means the degree of matching with a given nucleic acid sequence or amino acid sequence and may be expressed as a percentage (%). In the case of identity for nucleic acid sequences, for example, it may be determined using the algorithm BLAST by literature or FASTA by Pearson. Based on this algorithm BLAST, programs called BLASTN or BLASTX have been developed.

In one specific embodiment, the *E. coli* Nissle microorganism-derived lysine decarboxylase enzyme protein may comprise the amino acid sequence of SEQ ID NO: 1, or have an amino acid sequence having sequence identity of 90% or more, sequence identity of 95% or more, sequence identity of 97% or more, sequence identity of 99% or more, sequence identity of 99.5% or more, or sequence identity of 99.9% or more to the sequence, and have the pentamethylenediamine production activity. A polynucleotide encoding the *E. coli* Nissle microorganism-derived lysine decarboxylase enzyme protein may comprise the nucleic acid sequence of SEQ ID NO: 2, or have a nucleic acid sequence having sequence identity of 90% or more, sequence identity of 95% or more, sequence identity of 97% or more, sequence identity of 99% or more, sequence identity of 99.5% or more, or sequence identity of 99.9% or more to the sequence.

The recombinant microorganism may be an *Escherichia* sp. microorganism (specifically, *E. coli* Nissle, *E. coli* WL3110, *E. coli* XL1-Blue, *E. coli* BL21 (DE3)), a *Corynebacterium* sp. microorganism (specifically, *Corynebacterium glutamicum*) or a *Saccharomyces* sp. microorganism (specifically, *Saccharomyces cerevisiae*), but not limited thereto.

Other embodiment provides a recombinant vector, comprising a polynucleotide encoding *E. coli* Nissle microorganism-derived lysine decarboxylase enzyme protein.

A polynucleotide encoding the *E. coli* Nissle microorganism-derived lysine decarboxylase enzyme protein may be LdcC gene and/or CadA gene, but not limited thereto.

A microorganism comprising the recombinant vector or introduced with the recombinant vector may have pentamethylenediamine (PMDA) production activity, but not limited thereto.

In the present description, "vector" refers to any medium for cloning and/or transferring of a base into a host cell. The vector may be a replicon to which another DNA fragment binds to bring replication of the bound fragment. "Replicon" may mean any genetic unit (for example, plasmid, phage, cosmid, chromosome, virus), which functions as an autologous unit of DNA replication in vivo, i.e., is capable of replicating by its own regulation. In the present invention, the vector is not particularly limited as long as it is replicable in a host, and any vector known in the art may be used.

The vector used for construction of the recombinant vector, specifically, the vector used for introducing the polynucleotide encoding the *E. coli* Nissle microorganism-derived lysine decarboxylase enzyme protein into a microorganism may be a plasmid, a cosmid, a virus, and/or a bacteriophage in its natural or recombinant form. For example, as a phage vector or cosmid vector, pWE15, M13, λEMBL3, λEMBL4, λFIXII, λDASHII, λZAPII, λgt10, λgt11, Charon4A, and/or Charon21A and the like may be used, and as a plasmid vector, pKE vector, pHCMS vector, pHCP vector, pCES vector, pCG vector, pDZ vector, pBR-based, pUC-based, pBluescriptII-based, pGEM-based, pTZ-based, pCL-based, and/or pET-based and the like may be used. The available vector is not particularly limited, and a known expression vector may be used. In one embodiment, the vector may make a gene inserted into the vector and delivered to be irreversibly fused into the genome of a host cell so that gene expression can be stably sustained for a long period of time. Such a vector may comprise transcription and translation expression regulation sequences that allow the corresponding gene to be expressed in a selected host. As the expression regulation sequences, any operator sequence and/or a sequence regulating termination of transcription and translation for regulating transcription may be comprised. In one embodiment, a start codon and a stop codon may be generally considered a part of a nucleic acid sequence encoding a target protein, and they should exhibit an effect in a subject when a genetic construct is administered, and may be in frame with the coding sequence. In addition, in case of replicable expression vector, it may comprise a replication origin. Besides, an enhancer, a non-translated region at the 3' end of a targeted gene, a selective marker (for example, antibiotic resistant marker), and/or a replicable unit and the like may be appropriately comprised. The vector may be self-replicating or may be integrated into host genome DNA. According to one embodiment, each component in the vector should be operably linked to each other, and the linkage of these component sequences may be performed by ligation at a convenient restriction enzyme site, and if no such site is present, it may be performed using a synthetic oligonucleotide adaptor or linker according to a conventional method.

### Composition for producing pentamethylenediamine

Other embodiment of the present description provides a composition for producing pentamethylenediamine, comprising at least one selected from the group consisting of the recombinant microorganism, *E*. *coli* Nissle microorganism-derived lysine decarboxylase enzyme protein, the polynucleotide encoding the enzyme protein, and the recombinant vector.

The recombinant microorganism may refer to at least one selected from the group consisting of the recombinant microorganism itself (microbial cell), and culture, lysate and extract thereof, but not limited thereto.

The composition may further comprise a substrate. The substrate may be comprised at a concentration of 50 to 500 g/L, 50 to 400 g/L, 50 to 350 g/L, 50 to 320 g/L, 50 to 300 g/L, 50 to 250 g/L, 50 to 220 g/L, 50 to 200 g/L, 50 to 150 g/L, 50 to 120 g/L, 50 to 100 g/L, 80 to 500 g/L, 80 to 400 g/L, 80 to 350 g/L, 80 to 320 g/L, 80 to 300 g/L, 80 to 250 g/L, 80 to 220 g/L, 80 to 200 g/L, 80 to 150 g/L, 80 to 120 g/L, 80 to 100 g/L, 100 to 500 g/L, 100 to 400 g/L, 100 to 350 g/L, 100 to 320 g/L, 100 to 300 g/L, 100 to 250 g/L, 100 to 220 g/L, 100 to 200 g/L, 100 to 150 g/L, 100 to 120 g/L, 150 to 500 g/L, 150 to 400 g/L, 150 to 350 g/L, 150 to 320 g/L, 150 to 300 g/L, 150 to 250 g/L, 150 to 220 g/L, 150 to 200 g/L, 180 to 500 g/L, 180 to 400 g/L, 180 to 350 g/L, 180 to 320 g/L, 180 to 300 g/L, 180 to 250 g/L, 180 to 220 g/L, 180 to 200 g/L, 200 to 500 g/L, 200 to 400 g/L, 200 to 350 g/L, 200 to 320 g/L, 200 to 300 g/L, 200 to 250 g/L, 200 to 220 g/L, 250 to 500 g/L, 250 to 400 g/L, 250 to 350 g/L, 250 to 320 g/L, 250 to 300 g/L, 280 to 500 g/L, 280 to 400 g/L, 280 to 350 g/L, 280 to 320 g/L, 280 to 300 g/L, 300 to 500 g/L, 300 to 400 g/L, 300 to 350 g/L, 300 to 320 g/L, for example, 100 g/L, 200 g/L or 300 g/L, based on the total volume of the composition, but not limited thereto.

The substrate may be lysine, a carbon source, a nitrogen source, a phosphorus source, and/or a trace element component, but not limited thereto.

In the present description, "lysine" may refer to lysine alone and/or lysine comprising a salt thereof, and specifically, it may be at least one selected from the group consisting of lysine, lysine-HCl, lysine-H₂SO₄, and the like, but not limited thereto.

As the carbon source, glucose, glycerol, and/or molasses may be included. In addition, monosaccharides, oligosaccharides, polysaccharides, single carbon substrates, or mixtures thereof may be exemplified. For example, monosaccharides such as glucose and fructose; oligosaccharides such as sucrose, maltose or lactose; polysaccharides such as starch or cellulose; single carbon substrates such as methanol, formaldehyde or formate may be exemplified. In addition, lower alcohols such as ethanol, propanol, butanol and the like; polyols such as glycerol and the like; organic acids such as acetic acid, citric acid, succinic acid, tartaric acid, lactic acid, gluconic acid, and the like; fatty acids such as propionic acid, butanoic acid, pentanoic acid, hexanoic acid, heptanoic acid, octanoic acid, nonanoic acid, decanoic acid, undecanoic acid, dodecanoic acid, palmitic acid, stearic acid, linoleic acid, and the like; oils (specifically, vegetable oils) such as bean oil, sunflower oil, peanut oil, coconut oil and the like, but not limited thereto. These substances may be used individually or as a mixture.

As the nitrogen source, peptone, yeast extract, meat juice, malt extract, corn steep liquor, soybean meal and urea or inorganic compounds, for example, ammonium sulfate, ammonium chloride, ammonium phosphate, ammonium carbonate and ammonium nitrate may be exemplified, but not limited thereto. The nitrogen source may be also used individually or as a mixture.

As the phosphorus source, potassium dihydrogen phosphate or dipotassium hydrogen phosphate or corresponding sodium-containing salts may be exemplified, but not limited thereto. In addition, a growth medium may comprise metal salts such as magnesium sulfate or iron sulfate required for growth, or comprise essential growth substances such as amino acids and vitamins, but not limited thereto. The raw materials described above may be added to culture in a batch manner or in a continuous manner by an appropriate method during a culturing process.

The composition may further comprise PLP, but not limited thereto. The PLP may be comprised at a concentration of 1 to 1,000 µM, 1 to 800 µM, 1 to 600 µ M, 1 to 500 µM, 1 to 400 µM, 1 to 300 µM, 1 to 200 µM, 1 to 100 µM, 1 to 80 µM, 1 to 60 µM, 1 to 50 µM, 1 to 30 µM, 1 to 20 µM, 1 to 15 µM, 1 to 12 µM, 5 to 1,000 µM, 5 to 800 µM, 5 to 600 µM, 5 to 500 µM, 5 to 400 µM, 5 to 300 µM, 5 to 200 µM, 5 to 100 µM, 5 to 80 µM, 5 to 60 µM, 5 to 50 µM, 5 to 30 µM, 5 to 20 µM, 5 to 15 µM, 5 to 12 µM, 8 to 1,000 µM, 8 to 800 µM, 8 to 600 µM, 8 to 500 µM, 8 to 400 µM, 8 to 300 µM, 8 to 200 µM, 8 to 100 µM, 8 to 80 µM, 8 to 60 µM, 8 to 50 µM, 8 to 30 µM, 8 to 20 µM, 8 to 15 µM or 8 to 12 µM, for example, 10 µM, but not limited thereto.

### Method for producing pentamethylenediamine

Other embodiment of the present description provides a method for producing pentamethylenediamine, comprising reacting the recombinant microorganism with a substrate.

The recombinant microorganism may mean at least one selected from the group consisting of the recombinant microorganism itself (microbial cell), and culture, lysate and extract thereof, but not limited thereto.

The substate may react at a concentration of 50 to 500 g/L, 50 to 400 g/L, 50 to 350 g/L, 50 to 320 g/L, 50 to 300 g/L, 50 to 250 g/L, 50 to 220 g/L, 50 to 200 g/L, 50 to 150 g/L, 50 to 120 g/L, 50 to 100 g/L, 80 to 500 g/L, 80 to 400 g/L, 80 to 350 g/L, 80 to 320 g/L, 80 to 300 g/L, 80 to 250 g/L, 80 to 220 g/L, 80 to 200 g/L, 80 to 150 g/L, 80 to 120 g/L, 80 to 100 g/L, 100 to 500 g/L, 100 to 400 g/L, 100 to 350 g/L, 100 to 320 g/L, 100 to 300 g/L, 100 to 250 g/L, 100 to 220 g/L, 100 to 200 g/L, 100 to 150 g/L, 100 to 120 g/L, 150 to 500 g/L, 150 to 400 g/L, 150 to 350 g/L, 150 to 320 g/L, 150 to 300 g/L, 150 to 250 g/L, 150 to 220 g/L, 150 to 200 g/L, 180 to 500 g/L, 180 to 400 g/L, 180 to 350 g/L, 180 to 320 g/L, 180 to 300 g/L, 180 to 250 g/L, 180 to 220 g/L, 180 to 200 g/L, 200 to 500 g/L, 200 to 400 g/L, 200 to 350 g/L, 200 to 320 g/L, 200 to 300 g/L, 200 to 250 g/L, 200 to 220 g/L, 250 to 500 g/L, 250 to 400 g/L, 250 to 350 g/L, 250 to 320 g/L, 250 to 300 g/L, 280 to 500 g/L, 280 to 400 g/L, 280 to 350 g/L, 280 to 320 g/L, 280 to 300 g/L, 300 to 500 g/L, 300 to 400 g/L, 300 to 350 g/L, 300 to 320 g/L, for example, 100 g/L, 200 g/L or 300 g/L based on the volume of the at least one selected from the group consisting of culture of the recombinant microorganism, microbial cells of the recombinant microorganism, lysate of the microbial cells, and extract thereof, but not limited thereto.

The substrate may be a lysine complex, a carbon source, a nitrogen source, a phosphorus source, and/or a trace element component, but not limited thereto.

The method for producing pentamethylenediamine may further comprise reacting at least one selected from the group consisting of a recombinant microorganism, culture of the recombinant microorganism, microbial cells of the recombinant microorganism, lysate of the microbial cells, and extract thereof with PLP, but not limited thereto. The PLP may react at a concentration of 1 to 1,000 µM, 1 to 800 µM, 1 to 600 µM, 1 to 500 µM, 1 to 400 µM, 1 to 300 µM, 1 to 200 µM, 1 to 100 µM, 1 to 80 µM, 1 to 60 µM, 1 to 50 µM, 1 to 30 µM, 1 to 20 µM, 1 to 15 µM, 1 to 12 µM, 5 to 1,000 µM, 5 to 800 µM, 5 to 600 µM, 5 to 500 µM, 5 to 400 µM, 5 to 300 µM, 5 to 200 µM, 5 to 100 µM, 5 to 80 µM, 5 to 60 µM, 5 to 50 µM, 5 to 30 µM, 5 to 20 µM, 5 to 15 µM, 5 to 12 µM, 8 to 1,000 µM, 8 to 800 µM, 8 to 600 µM, 8 to 500 µM, 8 to 400 µM, 8 to 300 µM, 8 to 200 µM, 8 to 100 µM, 8 to 80 µM, 8 to 60 µM, 8 to 50 µM, 8 to 30 µM, 8 to 20 µM, 8 to 15 µM or 8 to 12 µM, for example, 10 µM, but not limited thereto.

The method for producing pentamethylenediamine may further comprise culturing the recombinant microorganism.

The method for producing pentamethylenediamine may further comprise separating and/or purifying pentamethylenediamine from culture (or, culture medium). The culture may be culture obtained by reacting at least one selected from the group consisting of a recombinant microorganism, culture of the recombinant microorganism, microbial cells of the recombinant microorganism, lysate of the microbial cells, and extract thereof with a substrate, but not limited thereto.

### [ADVANTAGEOUS EFFECTS]

The present invention can produce pentamethylenediamine using a recombinant microorganism comprising an *E. coli* Nissle-derived decarboxylase enzyme protein.

### [BRIEF DESCRIPTION OF DRAWINGS]

FIG. 1 shows a vector map of the pKE112 vector used for introduction of an *E. coli* Nissle-derived lysine decarboxylase enzyme gene.
FIG. 2 is a graph showing the results of confirming the pentamethylenediamine (PMDA) production activity of the microorganism according to introduction of an *E. coli* Nissle-derived lysine decarboxylase enzyme gene.
FIG. 3 is a graph showing the results of confirming the pentamethylenediamine (PMDA) production activity depending on the lysine decarboxylase enzyme-derived strain introduced into a recombinant microorganism or concentration of the used substrate.

### [MODE FOR INVENTION]

Hereinafter, the present invention will be described in detail by examples. However, the following examples illustrate the present invention only, but the present invention is not limited by the following examples.

### Examples 1 and 2. Preparation of recombinant microorganisms comprising E. coli Nissle-derived lysine decarboxylase enzyme gene

In order to confirm the activity of production of pentamethylenediamine (PMDA) of *E. coli* Nissle-derived lysine decarboxylase (LDC), a lysine decarboxylase enzyme gene was cloned.

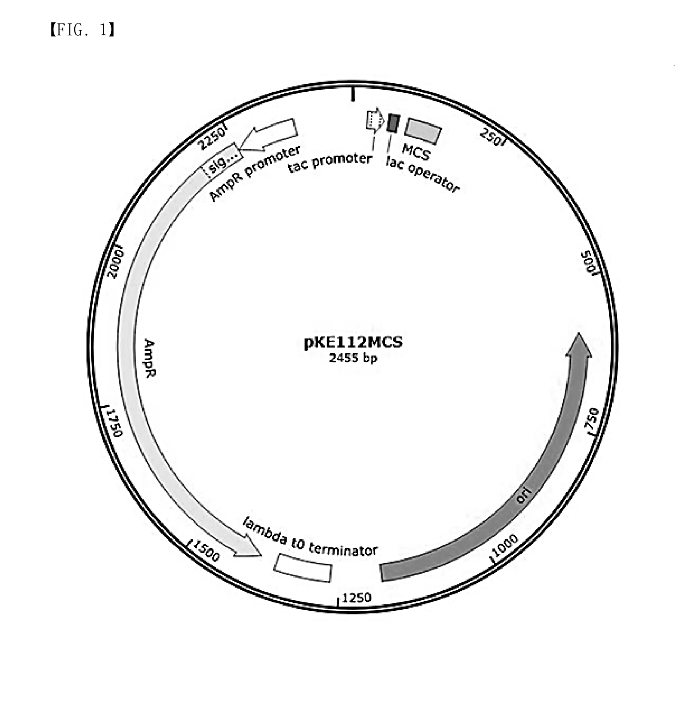

Specifically, in order to insert an *E. coli* Nissle-derived lysine decarboxylase enzyme (NCBI Accession Number: AXY47745.1) gene expressed by a *ldcC* gene (lysine decarboxylase *ldcC*) into pKE112 vector, pKE112 was digested using restriction enzymes KpnI/HindIII (New England Biolabs). The sequence information of the used restriction enzymes was shown in Table 1 below. The vector map of the pKE112 vector was shown in FIG. 1.

**[Table 1]**

| Type | Sequence (5' -> 3') | SEQ ID NO |
|---|---|---|
| KpnI | GGTACC | 7 |
| HindIII | AAGCTT | 8 |
| BamHI | GGATCC | 9 |
| SbfI | CCTGCAGG | 10 |

The *E. coli* Nissle-derived lysine decarboxylase was cloned by a process of decomposition using KpnI/HindIII and ligation to the pKE112 vector (pKE112_EcNLdcC).

The information of the amino acid sequence (SEQ ID NO: 1) and gene sequence (SEQ ID NO: 2) of the *E. coli* Nissle-derived lysine decarboxylase enzyme was shown in Table 2 below.

**[Table 2]**

| Type | Sequence (Amino acid sequence: N -> C; Nucleic acid sequence: 5' -> 3') | SEQ ID NO |
|---|---|---|
| EcNLdcC_AA | MNIIAIMGPHGVFYKDEPIKELESALVAQGFQIIWPQNSVDLLKFIEHNPRICGVIFDWDEYSLD LCSDINQLNEYLPLYAFINTHSTMDVSVQDMRMALWFFEYALGQAEDIAIRMRQYTNEYLDNITP PFTKALFTYVKERKYTFCTPGHMGGTAYQKSPVGCLFYDFFGGNTLKADVSISVTELGSLLDHTG PHLEAEEYIARTFGAEQSYIVTNGTSTSNKIVGMYAAPSGSTLLIDRNCHKSLAHLLMMNDVVPV WLKPTRNALGILGGIPRGEFTRDSIEEKVAATTQAQWPVHAVITNSTYDGLLYNTDWIKQTLDVP SIHFDSAWVPYTHFHPIYQGKSGMSGERVAGKVIFETQSTHKMLAALSQASLIHIKGEYDEEAFN EAFMMHTTTSPSYPIVASVETAAAMLRGNPGKRLINRSVERALHFRKEVQRLREESDGWFFDIWQ PPQVDEAECWPVAPGEQWHGFSDADANHMFLDPVKVTILTPGMDEQGNMSEEGIPAALVAKFLDE RGIVVEKTGPYNLLFLFSIGIDKTKAMGLLRGLTEFKRSYDLNLRIKNMLPDLYAEDPDFYRNMR IQDLAQGIHKLIRKHDLPGLMLRAFDTLPEMIMTPHQAWQRQIKGEVETIALEQLVGRVSANMIL PYPPGVPLLMPGEMLTEESRTVLDFLLMLCSVGQHYPGFETDIHGAKQDEDGVYRVRVLKMAG | 1 |
| *EcN1dcC_gene* | ATGAACATCATTGCCATTATGGGACCGCATGGCGTCTTTTATAAAGATGAGCCCATCAAAGAACT GGAGTCGGCGCTGGTGGCGCAAGGCTTTCAGATTATCTGGCCACAAAACAGCGTTGATTTGCTGA AGTTTATCGAACATAACCCACGAATTTGCGGCGTGATTTTTGACTGGGATGAGTACAGTCTCGAT TTATGTAGCGATATCAATCAACTTAATGAATATCTCCCGCTTTATGCCTTCATCAACACCCACTC | 2 |
| | GACGATGGATGTCAGCGTGCAGGATATGCGGATGGCGCTCTGGTTTTTTGAATATGCGCTGGGGC AGGCGGAAGATATCGCCATTCGTATGCGTCAGTACACCAACGAATATCTTGATAACATTACGCCG CCGTTCACGAAAGCCTTGTTTACCTACGTCAAAGAGCGGAAGTACACCTTTTGTACGCCGGGGCA TATGGGCGGCACCGCATATCAAAAAAGCCCGGTTGGCTGTCTGTTTTATGATTTTTTCGGCGGGA ATACCCTCAAGGCGGACGTCTCTATTTCGGTCACCGAGCTTGGTTCGTTGCTCGACCACACCGGG CCACACCTGGAAGCGGAAGAGTACATCGCGCGGACGTTTGGCGCGGAACAGAGTTATATCGTTAC CAACGGAACATCGACGTCGAACAAAATTGTGGGTATGTACGCCGCGCCATCCGGCAGTACGCTGT TGATCGACCGCAATTGTCATAAATCGCTGGCGCATCTGTTGATGATGAACGATGTAGTGCCAGTC TGGCTGAAACCGACGCGTAATGCGTTGGGGATTCTGGGTGGGATCCCGCGCGGTGAATTTACTCG CGACAGCATCGAAGAGAAAGTCGCTGCCACCACGCAGGCACAATGGCCGGTTCATGCGGTGATCA CCAACTCCACCTATGATGGCTTGCTCTACAACACCGACTGGATCAAACAGACGCTGGATGTCCCG TCGATTCACTTCGATTCTGCCTGGGTGCCGTACACCCATTTTCATCCAATCTACCAGGGTAAAAG TGGTATGAGCGGCGAGCGTGTTGCAGGAAAAGTGATCTTCGAAACGCAGTCGACCCACAAAATGC TGGCGGCGTTATCGCAGGCGTCGCTGATCCACATTAAAGGTGAGTATGACGAAGAGGCGTTTAAC GAAGCCTTTATGATGCATACCACCACCTCGCCCAGTTATCCCATTGTTGCTTCTGTTGAGACGGC GGCGGCGATGTTGCGTGGTAATCCGGGCAAACGGCTGATTAACCGTTCAGTAGAACGAGCTCTGC ATTTTCGCAAAGAGGTCCAGCGGCTGCGGGAAGAGTCTGACGGCTGGTTTTTCGATATCTGGCAA CCGCCGCAGGTGGATGAAGCCGAATGCTGGCCCGTTGCGCCTGGCGAACAGTGGCACGGCTTTAG CGATGCGGATGCCAATCACATGTTTCTCGATCCGGTTAAAGTCACTATTTTGACACCGGGGATGG ACGAGCAGGGCAATATGAGCGAGGAGGGGATCCCGGCGGCGCTGGTGGCAAAATTCCTCGACGAA CGTGGGATCGTAGTAGAGAAAACCGGCCCTTATAACCTGCTGTTTCTCTTTAGTATTGGCATCGA TAAAACCAAAGCAATGGGATTATTGCGTGGGTTGACGGAATTTAAGCGCTCTTACGATCTCAACC TGCGGATCAAAAATATGCTGCCCGATCTCTATGCAGAAGATCCCGATTTCTACCGCAATATGCGT ATTCAGGATCTGGCGCAAGGGATCCATAAGCTGATTCGTAAACACGATCTTCCCGGTTTGATGTT GCGGGCATTTGATACCTTGCCGGAGATGATCATGACGCCACATCAGGCATGGCAGAGACAGATTA AAGGCGAAGTAGAAACCATTGCGCTGGAACAACTGGTCGGTAGAGTATCGGCAAATATGATCCTG CCTTATCCACCCGGCGTACCGCTGCTGATGCCGGGAGAAATGCTTACCGAAGAGAGCCGCACGGT ACTCGATTTTCTACTGATGCTTTGTTCCGTAGGGCAACATTACCCCGGTTTTGAAACGGATATTC ACGGCGCGAAACAGGACGAAGACGGTGTTTACCGCGTACGAGTCCTAAAAATGGCAGGATAA | |

The cloned pKE112_EcNLdcC vector was transformed into an *E. coli* Nissle microorganism (*E. coli* Nissle 1917 microorganism used in Hwang, In Young, et al. "Engineered probiotic Escherichia coli can eliminate and prevent Pseudomonas aeruginosa gut infection in animal models." Nature communications 8.1 (2017): 15028. Lan, Yi-Jun, et al. "Development of Escherichia coli Nissle 1917 derivative by CRISPR/Cas9 and application for gamma-aminobutyric acid (GABA) production in antibiotic-free system." Biochemical Engineering Journal 168 (2021): 107952.) (Example 1) and an *E. coli* WL3110 microorganism (WL3110 microorganism used in Korean Patent Publication No. 2009-0018781) (Example 2), respectively, by a heat shock method.

### Comparative Example 1. Preparation of microorganism in which lysine decarboxylase enzyme gene is not transformed

The *E. coli* Nissle microorganism used in Example 1 in which the lysine decarboxylase enzyme gene was not transformed was prepared (Comparative Example 1).

### Comparative Examples 2 and 3. Preparation of recombinant microorganisms comprising wild-type E. coli-derived lysine decarboxylase enzyme gene

In order to confirm the pentamethylenediamine production activity of wild-type *E. coli*-derived lysine decarboxylase expressed by a *cadA* (lysine decarboxylase *cada*) gene (NCBI accession number: UDE09507.1) and wild-type *E. coli*-derived lysine decarboxylase expressed by a *ldcC* gene (NCBI accession number: UDE09932.1), the lysine decarboxylase enzyme genes were cloned.

Specifically, in order to transform the lysine decarboxylase enzyme gene expressed by the *cadA* gene into the *E. coli* WL3110, it was cloned into the pKE112 vector by substantially the same method as for the pKE112_EcNLdcC except for using KpnI/BamHI (New England Biolabs) as restriction enzymes (pKE112_EcCadA). In addition, the lysine decarboxylase enzyme gene expressed by the *ldcC* gene was also cloned into the pKE112 vector by substantially the same method as for the pKE112_EcNLdcC except for using KpnI/SbfI (New England Biolabs) as restriction enzymes (pKE112_EcLdcC). The sequence information of the used restriction enzymes shown in Table 1 above.

The information of the amino acid sequence (SEQ ID NO: 3) and gene sequence (SEQ ID NO: 4) of the wild-type *E. coli*-derived lysine decarboxylase enzyme expressed by the *cadA* gene, and the information of the amino acid sequence (SEQ ID NO: 5) and gene sequence (SEQ ID NO: 6) of the wild-type *E. coli-derived* lysine decarboxylase enzyme expressed by the *ldcC* gene were shown in Table 3 below.

**[Table 3]**

| Type | Sequence (Amino acid sequence: N -> C; Nucleic acid sequence: 5' -> 3') | SEQ ID NO |
|---|---|---|
| EcCadA_AA | MNVIAILNHMGVYFKEEPIRELHRALERLNFQIVYPNDRDDLLKLIENNARLCGVIFDWDKYNLELCEEISKMN ENLPLYAFANTYSTLDVSLNDLRLQISFFEYALGAAEDIANKIKQTTDEYINTILPPLTKALFKYVREGKYTFC TPGHMGGTAFQKSPVGSLFYDFFGPNTMKSDISISVSELGSLLDHSGPHKEAEQYIARVFNADRSYMVTNGTST ANKIVGMYSAPAGSTILIDRNCHKSLTHLMMMSDVTPIYFRPTRNAYGILGGIPQSEFQHATIAKRVKETPNAT WPVHAVITNSTYDGLLYNTDFIKKTLDVKSIHFDSAWVPYTNFSPIYEGKCGMSGGRVEGKVIYETQSTHKLLA AFSQASMIHVKGDVNEETFNEAYMMHTTTSPHYGIVASTETAAAMMKGNAGKRLINGSIERAIKFRKEIKRLRT ESDGWFFDVWQPDHIDTTECWPLRSDSTWHGFKNIDNEHMYLDPIKVTLLTPGMEKDGTMSDFGIPASIVAKYL DEHGIVVEKTGPYNLLFLFSIGIDKTKALSLLRALTDFKRAFDLNLRVKNMLPSLYREDPEFYENMRIQELAQN IHKLIVHHNLPDLMYRAFEVLPTMVMTPYAAFQKELHGMTEEVYLDEMVGRINANMILPYPPGVPLVMPGEMIT EESRPVLEFLQMLCEIGAHYPGFETDIHGAYRQADGRYTVKVLKEESKK | 3 |
| *EccadA_gene* | ATGAACGTTATTGCAATATTGAATCACATGGGGGTTTATTTTAAAGAAGAACCCATCCGTGAACTTCATCGCGC GCTTGAACGTCTGAACTTCCAGATTGTTTACCCGAACGACCGTGACGACTTATTAAAACTGATCGAAAACAATG CGCGTCTGTGCGGCGTTATTTTTGACTGGGATAAATATAATCTCGAGCTGTGCGAAGAAATTAGCAAAATGAAC GAGAACCTGCCGTTGTACGCGTTCGCTAATACGTATTCCACTCTCGATGTAAGCCTGAATGACCTGCGTTTACA GATTAGCTTCTTTGAATATGCGCTGGGTGCTGCTGAAGATATTGCTAATAAGATCAAGCAGACCACTGACGAAT ATATCAACACTATTCTGCCTCCGCTGACTAAAGCACTGTTTAAATATGTTCGTGAAGGTAAATATACTTTCTGT ACTCCTGGTCACATGGGCGGTACTGCATTCCAGAAAAGCCCGGTAGGTAGCCTGTTCTATGATTTCTTTGGTCC | 4 |
| | GAATACCATGAAATCTGATATTTCCATTTCAGTATCTGAACTGGGTTCTCTGCTGGATCACAGTGGTCCACACA AAGAAGCAGAACAGTATATCGCTCGCGTCTTTAACGCAGACCGCAGCTACATGGTGACCAACGGTACTTCCACT GCGAACAAAATTGTTGGTATGTACTCTGCTCCAGCAGGCAGCACCATTCTGATTGACCGTAACTGCCACAAATC GCTGACCCACCTGATGATGATGAGCGATGTTACGCCAATCTATTTCCGCCCGACCCGTAACGCTTACGGTATTC TTGGTGGTATCCCACAGAGTGAATTCCAGCACGCTACCATTGCTAAGCGCGTGAAAGAAACACCAAACGCAACC TGGCCGGTACATGCTGTAATTACCAACTCTACCTATGATGGTCTGCTGTACAACACCGACTTCATCAAGAAAAC ACTGGATGTGAAATCCATCCACTTTGACTCCGCGTGGGTGCCTTACACCAACTTCTCACCGATTTACGAAGGTA AATGCGGTATGAGCGGTGGCCGTGTAGAAGGGAAAGTGATTTACGAAACCCAGTCCACTCACAAACTGCTGGCG GCGTTCTCTCAGGCTTCCATGATCCACGTTAAAGGTGACGTAAACGAAGAAACCTTTAACGAAGCCTACATGAT GCACACCACCACTTCTCCGCACTACGGTATCGTGGCGTCCACTGAAACCGCTGCGGCGATGATGAAAGGCAATG CAGGTAAGCGTCTGATCAACGGTTCTATTGAACGTGCGATCAAATTCCGTAAAGAGATCAAACGTCTGAGAACG GAATCTGATGGCTGGTTCTTTGATGTATGGCAGCCGGATCATATCGATACGACTGAATGCTGGCCGCTGCGTTC TGACAGCACCTGGCACGGCTTCAAAAACATCGATAACGAGCACATGTATCTTGACCCGATCAAAGTCACCCTGC TGACTCCGGGGATGGAAAAAGACGGCACCATGAGCGACTTTGGTATTCCGGCCAGCATCGTGGCGAAATACCTC GACGAACATGGCATCGTTGTTGAGAAAACCGGTCCGTATAACCTGCTGTTCCTGTTCAGCATCGGTATCGATAA GACCAAAGCACTGAGCCTGCTGCGTGCTCTGACTGACTTTAAACGTGCGTTCGACCTGAACCTGCGTGTGAAAA ACATGCTGCCGTCTCTGTATCGTGAAGATCCTGAATTCTATGAAAACATGCGTATTCAGGAACTGGCTCAGAAT ATCCACAAACTGATTGTTCACCACAATCTGCCGGATCTGATGTATCGCGCATTTGAAGTGCTGCCGACGATGGT AATGACTCCGTATGCTGCATTCCAGAAAGAGCTGCACGGTATGACCGAAGAAGTTTACCTCGACGAAATGGTAG GTCGTATTAACGCCAATATGATCCTTCCGTACCCGCCGGGAGTTCCTCTGGTAATGCCGGGTGAAATGATCACC GAAGAAAGCCGTCCGGTTCTGGAGTTCCTGCAGATGCTGTGTGAAATCGGCGCTCACTATCCGGGCTTTGAAAC CGATATTCACGGTGCATACCGTCAGGCTGATGGCCGCTATACCGTTAAGGTATTGAAAGAAGAAAGCAAAAAAT AA | |
| EcLdcC_AA | MNIIAIMGPHGVFYKDEPIKELESALVAQGFQIIWPQNSVDLLKFIEHNPRICGVIFDWDEYSLDLCSDINQLN EYLPLYAFINTHSTMDVSVQDMRMALWFFEYALGQAEDIAIRMRQYTDEYLDNITPPFTKALFTYVKERKYTFC TPGHMGGTAYQKSPVGCLFYDFFGGNTLKADVSISVTELGSLLDHTGPHLEAEEYIARTFGAEQSYIVTNGTST SNKIVGMYAAPSGSTLLIDRNCHKSLAHLLMMNDVVPVWLKPTRNALGILGGIPRREFTRDSIEEKVAATTQAQ WPVHAVITNSTYDGLLYNTDWIKQTLDVPSIHFDSAWVPYTHFHPIYQGKSGMSGERVAGKVIFETQSTHKMLA ALSQASLIHIKGEYDEEAFNEAFMMHTTTSPSYPIVASVETAAAMLRGNPGKRLINRSVERALHFRKEVQRLRE ESDGWFFDIWQPPQVDEAECWPVAPGEQWHGFNDADADHMFLDPVKVTILTPGMDEQGNMSEEGIPAALVAKFL DERGIVVEKTGPYNLLFLFSIGIDKTKAMGLLRGLTEFKRSYDLNLRIKNMLPDLYAEDPDFYRNMRIQDLAQG IHKLIRKHDLPGLMLRAFDTLPEMIMTPHQAWQRQIKGEVETIALEQLVGRVSANMILPYPPGVPLLMPGEMLT KESRTVLDFLLMLCSVGQHYPGFETDIHGAKQDEDGVYRVRVLKMAG | 5 |
| *EcldcC*_gene | ATGAACATCATTGCCATTATGGGACCGCATGGCGTCTTTTATAAAGATGAGCCCATCAAAGAACTGGAGTCGGC GCTGGTGGCGCAAGGCTTTCAGATTATCTGGCCACAAAACAGCGTTGATTTGCTGAAATTTATCGAGCATAACC CTCGAATTTGCGGCGTGATTTTTGACTGGGATGAGTACAGTCTCGATTTATGTAGCGATATCAATCAGCTTAAT GAATATCTCCCGCTTTATGCCTTCATCAACACCCACTCGACGATGGATGTCAGCGTGCAGGATATGCGGATGGC GCTCTGGTTTTTTGAATATGCGCTGGGGCAGGCGGAAGATATCGCCATTCGTATGCGTCAGTACACCGACGAAT ATCTTGATAACATTACACCGCCGTTCACGAAAGCCTTGTTTACCTACGTCAAAGAGCGGAAGTACACCTTTTGT ACGCCGGGGCATATGGGCGGCACCGCATATCAAAAAAGCCCGGTTGGCTGTCTGTTTTATGATTTTTTCGGCGG GAATACTCTTAAGGCTGATGTCTCTATTTCGGTCACCGAGCTTGGTTCGTTGCTCGACCACACCGGGCCACACC TGGAAGCGGAAGAGTACATCGCGCGGACTTTTGGCGCGGAACAGAGTTATATCGTTACCAACGGAACATCGACG TCGAACAAAATTGTGGGTATGTACGCCGCGCCATCCGGCAGTACGCTGTTGATCGACCGCAATTGTCATAAATC GCTGGCGCATCTGTTGATGATGAACGATGTAGTGCCAGTCTGGCTGAAACCGACGCGTAATGCGTTGGGGATTC TTGGTGGGATCCCGCGCCGTGAATTTACTCGCGACAGCATCGAAGAGAAAGTCGCTGCTACCACGCAAGCACAA TGGCCGGTTCATGCGGTGATCACCAACTCCACCTATGATGGCTTGCTCTACAACACCGACTGGATCAAACAGAC | 6 |
| | GCTGGATGTCCCGTCGATTCACTTCGATTCTGCCTGGGTGCCGTACACCCATTTTCATCCGATCTACCAGGGTA AAAGTGGTATGAGCGGCGAGCGTGTTGCGGGAAAAGTGATCTTCGAAACGCAATCGACCCACAAAATGCTGGCG GCGTTATCGCAGGCTTCGCTGATCCACATTAAAGGCGAGTATGACGAAGAGGCCTTTAACGAAGCCTTTATGAT GCATACCACCACCTCGCCCAGTTATCCCATTGTTGCTTCGGTTGAGACGGCGGCGGCGATGCTGCGTGGTAATC CGGGCAAACGGCTGATTAACCGTTCAGTAGAACGAGCTCTGCATTTTCGCAAAGAGGTCCAGCGGCTGCGGGAA GAGTCTGACGGTTGGTTTTTCGATATCTGGCAACCGCCGCAGGTGGATGAAGCCGAATGCTGGCCCGTTGCGCC TGGCGAACAGTGGCACGGCTTTAACGATGCGGATGCCGATCATATGTTTCTCGATCCGGTTAAAGTCACTATTT TGACACCGGGGATGGACGAGCAGGGCAATATGAGCGAGGAGGGGATCCCGGCGGCGCTGGTAGCAAAATTCCTC GACGAACGTGGGATCGTAGTAGAGAAAACCGGCCCTTATAACCTGCTGTTTCTCTTTAGTATTGGCATCGATAA AACCAAAGCAATGGGATTATTGCGTGGGTTGACGGAATTCAAACGCTCTTACGATCTCAACCTGCGGATCAAAA ATATGCTACCCGATCTCTATGCAGAAGATCCCGATTTCTACCGCAATATGCGTATTCAGGATCTGGCACAAGGG ATCCATAAGCTGATTCGTAAACACGATCTTCCCGGTTTGATGTTGCGGGCATTCGATACTTTGCCGGAGATGAT CATGACGCCACATCAGGCATGGCAACGACAAATTAAAGGCGAAGTAGAAACCATTGCGCTGGAACAACTGGTCG GTAGAGTATCGGCAAATATGATCCTGCCTTATCCACCGGGCGTACCGCTGTTGATGCCTGGAGAAATGCTGACC AAAGAGAGCCGCACAGTACTCGATTTTCTACTGATGCTTTGTTCCGTCGGGCAACATTACCCCGGTTTTGAAAC GGATATTCACGGCGCGAAACAGGACGAAGACGGCGTTTACCGCGTACGAGTCCTAAAAATGGCGGGATAA | |

Each of the cloned pKE112_EcCadA (Comparative Example 2) and pKE112_EcLdcC (Comparative Example 3) vectors was transformed into the same microorganism as the *E. coli* WL3110 microorganism used in Example 2 by a heat shock method.

### Test example 1. Measurement of pentamethylenediamine production activity of recombinant microorganisms comprising E. coli Nissle-derived lysine decarboxylase enzyme genes

The microorganisms prepared in Example 1 and Comparative Example 1 were cultured in the MR medium (6.67 g/L KH₂PO₄, 4 g/L (NH₄)₂HPO₄, 0.8 g/L MgSO_{4·}7H₂O, 0.8 g/L citric acid, 5mL trace metal solution) comprising 20g/L glucose and 10mg/L thiamine-HCl at 30°C for 16 hours. During culturing the microorganism of Example 1, the ampicillin antibiotic was added at a concentration of 50 µg/mL, and microorganisms confirmed to be transformed were selected through screening of surviving microorganisms.

For the cultured microorganisms, centrifugation was performed under the conditions of 4°C, 4000 rpm, 15 minutes, and the cells obtained by this were diluted with distilled water at a concentration of OD₆₀₀=50, and lysine-HCl adjusted to pH 7.0 was added so that the final concentration was 200g/L, and additionally, they were divided into a group in which pyridoxal 5'-phoasphate (PLP) facilitating LDC enzyme activity was added at a concentration of 10 µM and a group in which PLP was not added, and the enzyme conversion reaction was performed at 37°C for 120 hours.

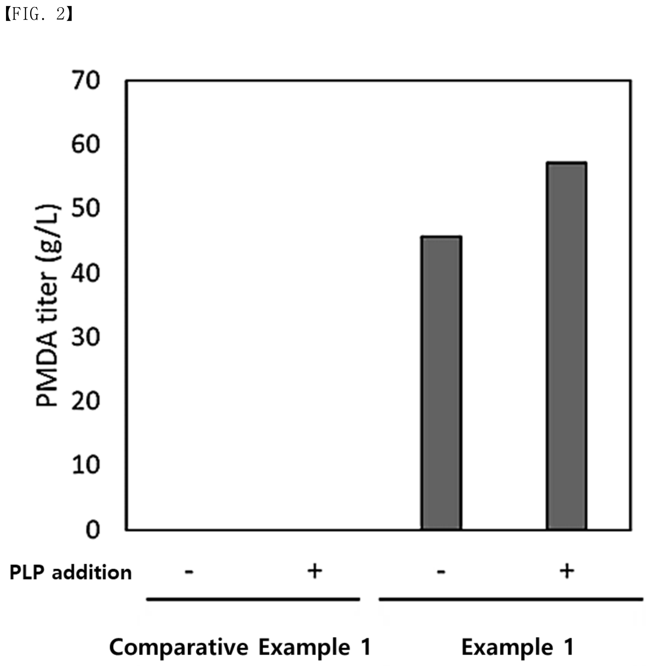

The final production concentration of pentamethylenediamine produced after conducting the enzyme conversion reaction (PMDA titer) was measured by HPLC after passing through a diethyl ethoxymethylenemalonate (DEEMM) reaction, and the results were shown in Table 4 and FIG. 2 below.

Specifically, HPLC equipped with Variable Wavelength Detector (VWD) and C18 column was used, and was eluted with Mobile phase A and mobile phase B. The flow rate was set to 1 mL/min and analysis was conducted at 284 nm.

**[Table 4]**

| Microorganism type | PLP addition | PMDA titer (g/L) |
|---|---|---|
| Example 1 | - | 45.7 |
| | + | 57.2 |
| Comparative Example 1 | - | 0 |
| | + | 0 |

As a result of confirming the final production concentration of pentamethylenediamine, it was confirmed that the microorganism in which the *E. coli* Nissle-derived lysine decarboxylase enzyme gene was transformed produced PMDA, but it was confirmed that the non-transformed *E. coli* Nissle microorganism did not produce PMDA. In addition, it was confirmed that the PMDA production was higher when PLP was added.

### Test example 2. Measurement of pentamethylenediamine production activity depending on lysine decarboxylase enzyme-derived strain and substrate concentration

The microorganisms of Example 2 and Comparative Examples 2, 3 were cultured by the substantially the same method as the culture method of the microorganism of Example 1 in Test Example 1 above.

For the cultured microorganisms, centrifugation was performed under the conditions of 4°C, 4000 rpm, 15 minutes, and the cells obtained through this were diluted with distilled water at a concentration of OD₆₀₀=50, and lysine-HCl adjusted to pH 7.0 was added so that the final concentration was 100, 200 or 300 g/L, and the enzyme conversion reaction was conducted at 37°C for 120 hours.

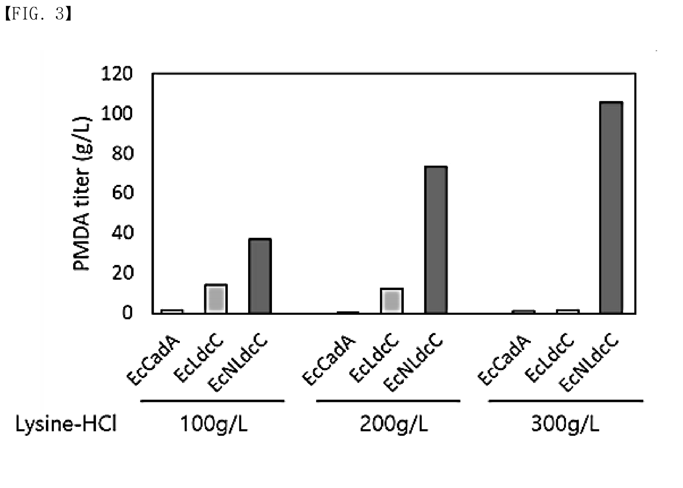

The final production concentration of pentamethylenediamine produced after conducting the enzyme conversion reaction (PMDA titer) was measured by the substantially the same method as Text Example 1 above, and the results were shown in Table 5 and FIG. 3 below.

**[Table 5]**

| Microorganism type | lysine-HCl final concentration (g/L) | PMDA titer (g/L) |
|---|---|---|
| Example 2 | 100 | 36.94 |
| | 200 | 73.13 |
| | 300 | 105.24 |
| Comparative Example 2 | 100 | 1.66 |
| | 200 | 0.26 |
| | 300 | 1.13 |
| Comparative Example 3 | 100 | 14.06 |
| | 200 | 12.29 |
| | 300 | 1.9 |

As a result of confirming the final production concentration of pentamethylenediamine, it was confirmed that the PMDA production of the microorganism in which the *E. coli* Nissle-derived lysine decarboxylase was transformed was higher than the microorganism in which the wild-type *E*. *coli-*derived lysine decarboxylase was transformed.

It was confirmed that in the microorganism in which the *E. coli* Nissle-derived lysine decarboxylase was transformed (Example 2), the PDMA production continued to increase as the lysine-HCl concentration increased.

In addition, as a result of confirming the PMDA production of Comparative Examples 2 and 3, which are microorganisms in which lysine decarboxylase expressed by the *cadA* and *ldcC* genes, respectively, was transformed, it was confirmed that the PMDA production of Comparative Example 3, which is the microorganism in which lysine decarboxylase expressed by the *ldcC* gene was transformed, was higher.

## Claims

1. A recombinant microorganism, comprising an *E. coli* Nissle microorganism-derived lysine decarboxylase (LDC) enzyme protein, or a polynucleotide encoding an *E. coli* Nissle microorganism-derived lysine decarboxylase enzyme protein, and
having production activity of pentamethylenediamine (PMDA).

2. The recombinant microorganism according to claim 1, wherein the polynucleotide encoding the *E. coli* Nissle microorganism-derived lysine decarboxylase enzyme protein is introduced.

3. The recombinant microorganism according to claim 1, wherein the *E. coli* Nissle microorganism-derived lysine decarboxylase enzyme protein comprises the amino acid sequence of SEQ ID NO: 1.

4. The recombinant microorganism according to claim 1, wherein the recombinant microorganism is an *Escherichia* sp. microorganism, a *Corynebacterium* sp. microorganism, or a *Saccharomyces* sp. microorganism.

5. The recombinant microorganism according to claim 1, wherein the lysine decarboxylase enzyme protein is expressed by *ldcC* gene.

6. A composition for producing pentamethylenediamine, comprising at least one selected from the group consisting of the recombinant microorganism of any one claim of claim 1 to claim 5, an *E. coli* Nissle microorganism-derived lysine decarboxylase enzyme protein, a polynucleotide encoding the enzyme protein, and a recombinant vector comprising the polynucleotide.

7. The composition for producing pentamethylenediamine according to claim 6, wherein the composition further comprises a substrate.

8. The composition for producing pentamethylenediamine according to claim 7, wherein the substrate is comprised at a concentration of 50 to 500 g/L based on the total volume of the composition.

9. The composition for producing pentamethylenediamine according to claim 7, wherein the substrate is at least one selected from the group consisting of lysine, lysine-HCl, lysine-H₂SO₄, glucose, sucrose, lactose, fructose, maltose, molasses, starch, and cellulose.

10. A method for producing pentamethylenediamine, comprising reacting the recombinant microorganism of any one claim of claim 1 to claim 5 with a substrate.

11. The method for producing pentamethylenediamine according to claim 10, wherein the substrate is reacted at a concentration of 50 to 500 g/L based on the volume of culture of the recombinant microorganism.

12. The method for producing pentamethylenediamine according to claim 10, wherein the substrate is at least one selected from the group consisting of lysine, lysine-HCl and lysine-H₂SO₄.
